# EUROPÄISCHE PATENTSCHRIFT

(11) **EP 1 996 261 B1**
(45) Veröffentlichungstag und Bekanntmachung des Hinweises auf die Patenterteilung: **18.11.2015**
(21) Anmeldenummer: 07722062.2
(22) Anmeldetag: 16.03.2007
(51) Int. Cl.: A61M 5/32

(54) **KANÜLE MIT EINEM SCHUTZGEHÄUSE**
CANNULA EQUIPPED WITH A PROTECTIVE HOUSING
CANULE COMPRENANT UN LOGEMENT DE PROTECTION

(30) Priorität: 21.03.2006 DE 102006013322
(43) Veröffentlichungstag der Anmeldung: 03.12.2008
(73) Patentinhaber: Sarstedt AG & Co., 51588 Nümbrecht (DE)
(72) Erfinder: SARSTEDT, Walter, 51588 Nümbrecht (DE)
(74) Vertreter: Grosse, Wolf-Dietrich Rüdiger
(86) Internationale Anmeldenummer: PCT/DE2007/000502
(87) Internationale Veröffentlichungsnummer: WO 2007/110043

(56) Entgegenhaltungen:
- EP-A- 0 381 577
- EP-A- 1 186 313
- EP-A- 1 602 327
- US-A1- 2002 156 424

## Beschreibung

Die Erfindung betrifft eine Kanüle, vorzugsweise mit einem Kanülenhalter für insbesondere eine Blutentnahmevorrichtung, die ein schwenkbares, bis auf eine zwischen einander gegenüberliegenden Seitenwänden vorgesehene, von mindestens einem an den Seitenwänden ausgeformten, nach innen trichterförmig aufeinander zu laufenden, dort einen aufweitbaren Trichterschlitz bildenden Lamellenpaar bereitgestellte Kanüleneintrittsöffnung umfangsgeschlossenes Schutzgehäuse für ein freies Kanülenende aufweist, wie durch die EP 1 186 313 A bekannt geworden.

Eine Kanüle mit einem Schutzgehäuse offenbart ebenfalls die US 2002/156 424 A. Dort sind an das Schutzgehäuse nach oben vorstehende Schwenkklappen angeformt, die beim Zusammendrücken die Gehäuseöffnung zum Ein- und Austritt der Nadel weiten.

Bei einer aus der EP 1 602 327 A1 bekannten Blutentnahmevorrichtung sind die freien Enden der Seitenwände des Schutzgehäuses einer insbesondere als Doppelkanüle ausgebildeten Nadel komplementär zu einem sich weitestgehend über die gesamte Gehäuselänge erstreckenden Kanülen-Einführtrichter ausgeformt.

Aus der US 5.490.841, DE 691 27 906 T2, DE 692 25 609 T2 und US 3.658.061 sind solche Blutentnahmevorrichtungen mit einem in vielfältigsten Varianten ausgebildeten Sicherheits- bzw. Schutzgehäuse bekannt. Solche Schutzgehäuse kommen auch für die Kanülen von Injektionsspritzen zur Anwendung. Damit der Benutzer oder ein Dritter nicht mit der Kanüle bzw. daran verbliebenen Bluttropfen in Berührung kommt sowie auch zur Vermeidung von Stichverletzungen, wird das Schutzgehäuse nach Beendigung der Blutentnahme fluchtend zur Kanüle verschwenkt, die danach von dem Schutzgehäuse aufgenommen wird. Das Schutzgehäuse weist als Kanüleneintrittsöffnung einen langgestreckten, offenen Schlitz auf. Durch diesen Schlitz tritt die Kanüle ein, wenn das längliche Schutzgehäuse in die mit der Kanüle fluchtende Position geschwenkt wird.

Im Schutzgehäuse selbst sind Zurückhaltemittel vorgesehen, um zu verhindern, dass die eingeschwenkte Kanüle aus dem Schutzgehäuse ungewollt, selbsttätig wieder austritt. Diese Zurückhaltemittel sind in Form von Haken ausgebildet, die in dem von den Schutzgehäusewänden eingeschlossenen Kanal und somit im Inneren des Schutzgehäuses an dessen Bodenwand bzw. im Bereich des Bodens angeordnet sind. Bei diesen Blutentnahmevorrichtungen ist das Schutzgehäuse über eine Lasche und einen Klemmring entweder stationär oder drehbar an dem Kanülenhalter befestigt.

Die bekannten Schutzgehäuse weisen den Nachteil auf, dass die Haken als Zurückhaltemittel nicht sicher verhindern können, dass die Kanüle selbsttätig doch wieder austritt. Es ist nämlich nicht ausgeschlossen, dass die Kanüle seitlich entlang der freien Hakenenden wieder aus dem Schutzgehäuse herausgleitet. Dazu trägt, z.B. bei US 5.490.841, wesentlich bei, dass sich der Einführungsschlitz noch weiter öffnet, wenn von der Schlitzseite her Druck auf die zum leichteren Einführen der Kanüle flexiblen sich weitestgehend über die gesamte Gehäuselänge erstreckenden Lamellen ausgeübt wird, so dass sich die Kanüleneintrittsöffnung erweitert bzw. aufspreizt. Die Kanüle/Nadel kann dann ohne weiteres unerwünscht aus dem Schutzgehäuse austreten. Damit liegt weiterhin das Problem vor, dass mitunter sogar auch kontaminierte Flüssigkeit austreten kann. Abgesehen davon ist ein Schutzgehäuse mit zusätzlichen Zurückhaltemitteln bzw. Haken nur aufwendig herzustellen und macht zusätzliche Gehäusedurchbrechungen (vgl. die vorgenannte DE 691 27 906 T2 und DE 692 25 609 T2) erforderlich. Schließlich setzt das geradezu notwendige Einfädeln des freien Kanülenendes in die Hakenmittel bei einer Bedienungsperson eine gewisse Geschicklichkeit und besondere Aufmerksamkeit voraus.

Der Erfindung liegt daher die Aufgabe zugrunde, eine gattungsgemäße Kanüle ohne diese Nachteile zu schaffen und insbesondere mit einer hohen Sicherheitsfunktion für den Benutzer bereitzustellen.

Diese Aufgabe wird erfindungsgemäß dadurch gelöst, dass das Schutzgehäuse aus zwei konzentrisch ineinander gesteckten Gehäuseteilen besteht, wobei ein Innengehäuse das Lamellenpaar aufweist und ein das Innengehäuse umschließendes Außengehäuse als eine in Bezug auf das Innengehäuse weniger elastische Hülse ausgebildet ist. Die Erfindung löst sich somit von der vorgegebenen, üblichen einteiligen Bauweise eines Schutzgehäuses und ermöglicht durch das massivere, weitestgehend unelastische Außengehäuse, daß das wegen der flexiblen Lamellenpaare, wobei alternativ lediglich eine Lamelle flexibel zu sein brauchte, während die andere Lamelle starr sein könnte, notwendiger Weise aus elastischem, nachgiebigem Material bestehende Innengehäuse vor von außen aufgebrachten Druckkräften geschützt wird, so daß sich der Trichterschlitz nicht unerwünscht weiten kann. Hierbei ist es ganz gleich, ob das Innengehäuse mit dem Kanülenhalter verbunden ist und darüber das weniger elastische Außengehäuse aufgeschoben wird oder das Außengehäuse mit dem Kanülenhalter verbunden ist und in dieses dann das Innengehäuse eingeschoben wird.

Eine Ausgestaltung der Erfindung sieht vor, dass das Außengehäuse mit Kontaktflächen, ausgebildet vorteilhaft als Schrägflächen oder Radien, versehen ist, die bei äußerem Druck auf gegenüberliegende, komplementär vorgesehene Kontaktflächen des Innengehäuses eine auf den Trichterschlitz gerichtete Schließkraft einleiten. Hierbei kann von zwei ineinander gesteckten Gehäuseteilen das Innengehäuse das Lamellenpaar aufweisen und ein das Innengehäuse umschließendes Außengehäuse kann als eine in Bezug auf das Innengehäuse weniger elastische Hülse ausgebildet sein.

Hierbei empfiehlt es sich, daß das die Druckkraft ausübende bzw. einen unbeabsichtigten äußeren Druck weiterleitende Außengehäuse dort mit Schrägflächen oder dergleichen Kontaktflächen eine gezielte Krafteinleitung ermöglichenden Mitteln ausgebildet ist, wo es das Innengehäuse oben, an den freien Enden der Seitenwände mit den daran nach innen einander zugewandt ausgeformten Lamellen übergreift, die an komplementären Schrägflächen oder dergleichen Kontaktflächen des Innengehäuses anliegen. Der somit erreichte Flächenkontakt trägt zur definierten Krafteinleitung am Ort des Geschehens, nämlich im Bereich der und auf die Lamellen bei. Die über das Außengehäuse eingeleitete seitliche Druckkraft hält den Trichterschlitz sicher geschlossen, d.h. die freien Enden der aufeinander zu geführten flexiblen Lamellen kommen stets zur gegenseitigen Anlage

Außerdem ist der trichterförmigen Kanüleneintrittsöffnung des Innengehäuses durch das umschließende Außengehäuse eine Zugangsöffnung vorgelagert, die von die Seitenwände des Innengehäuses nach oben überkragenden, einander zugewandt ausgeformten Außengehäuse-Kopfstegen gebildet wird. Ein Kontakt mit dem Innengehäuse, insbesondere den Lamellen, ist ausgeschlossen, so dass die Sicherheitsfunktion nicht beeinträchtigt werden kann. Die Zugangsöffnungt erlaubt es nämlich nicht, dass beispw. ein Finger einer Bedienungsperson die Außengehäuse-Kopfstege passieren und Druck auf die Lamellen ausüben kann.

Weitere Merkmale und Einzelheiten der Erfindung ergeben sich aus den Ansprüchen und der nachfolgenden Beschreibung von in den Zeichnungen im Zusammenhang mit einer Blutentnahmevorrichtung dargestellten Ausführungsbeispielen der Erfindung. Es zeigen:
- Figur 1: als Einzelheit einer Blutentnahmevorrichtung in einer Gesamtansicht einen Kanülenhalter mit einem daran angeordneten, herkömmlichen Schutzgehäuse, vor dem Verschließen dargestellt;
- Figur 2: als Einzelheit einer Blutentnahmevorrichtung in einer Gesamtansicht einen Kanülenhalter mit daran angeordnetem, aus zwei Gehäuseteilen bestehendem Schutzgehäuse, vor dem Verschließen dargestellt;
- Figur 3: als Einzelheit der Figur 1 einen Querschnitt durch das Schutzgehäuse;
- Figur 4: als Einzelheit der Figur 2 mit vergrößertem Maßstab einen Querschnitt des Schutzgehäuses; und
- Figur 5: eine Abwandlung des in Fig. 4 dargestellten Schutzgehäuses.

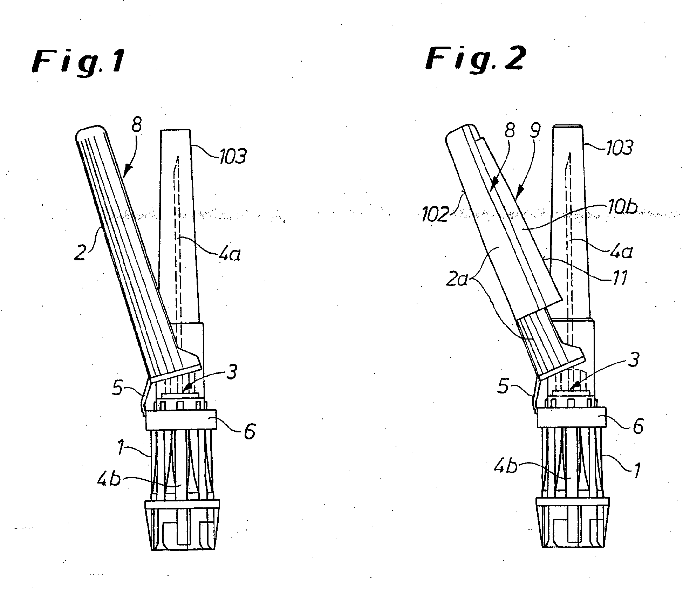

Ein in den Figuren 1 und 2 gezeigter, mit einem nicht dargestellten Blutentnahmeröhrchen verbindbarer Kanülenhalter 1 wird üblich im Zustand mit ausgeschwenktem bzw. abgeklapptem Schutzgehäuse 2, 102 geliefert, wobei ein freies Kanülenende 4a seiner Kanüle bzw. hier Doppelkanüle 3, deren unteres Kanülenende 4b ebenfalls eine angeschärfte. Spitze besitzt, durch eine separate Umhüllung 103 geschützt ist, die vor dem Gebrauch entfernt wird. Nach dem Gebrauch wird dann das freie Kanülenende 4a der Doppelkanüle 3 mit dem Schutzgehäuse 2 bzw. 102 umschlossen und von diesem eingekammert.

Das Schutzgehäuse 2 bzw. 102 ist im Ausführungsbeispiel über ein Band- oder Laschenscharnier 5 an einem Haltering 6 befestigt, der am oberen, dem freien Kanülenende 4a zugewandten Ende des Kanülenhalters 1 angeordnet ist.

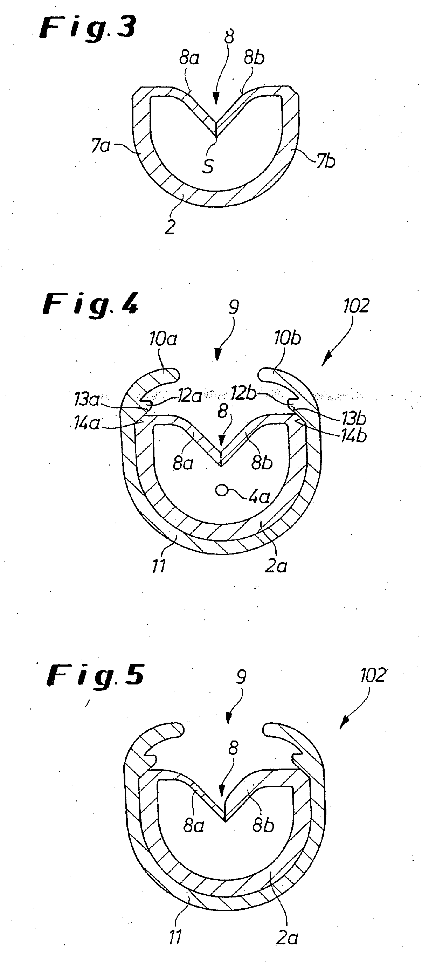

Das bekannte, durch Spritzgießen aus einem weichelastischen Kunststoff hergestellte Schutzgehäuse 2 besitzt eine von an den freien Enden ihrer Seitenwände 7a, 7b ausgeformeten, nach innen trichterförmig aufeinander zu laufenden und im Ausführungsbeispiel sich weitestgehend über die gesamte Gehäuselänge erstreckenden, flexiblen Lamellenpaaren 8a, 8b - im folgenden stets nur Lamellen genannt - gebildete Kanüleneintrittsöffnung 8. Im Mündungsbereich der flexiblen Lamellen 8a, 8b liegt ein Trichterschlitz S vor, wie in Fig. 3 dargestellt. Eine im Inneren des Schutzgehäuses 2 eingefangene Kanüle kann, wenn unbeabsichtigt eine Druckkraft auf die Lamellen aufgebracht wird, damit über den Trichterschlitz S und die Kanüleneintrittsöffnung 8 unerwünscht wieder aus dem Schutzgehäuse 2 herausgleiten.

Dieser Nachteil des unerwünschten Heraustretens des freien Kanülenendes 4a wird nach den Figuren 2 und 4 durch eine zweiteilige Ausführung des Schutzgehäuses 102 vermieden. Es besteht aus einem elastischen, die flexiblen Lamellen 8a, 8b aufweisenden Innengehäuse 2a und einem darauf aufgeschobenem Außengehäuse 11 aus einem gegenüber dem Innengehäuse 2a formstabileren, weit weniger elastischem Material. Im Ausführungsbeispiel ist das Innengehäuse 2a über das Band- oder Laschenscharnier 5 mit dem Haltering 6 des Kanülenhalters 1 verbunden; alternativ kann das Außengehäuse 11 mit dem Haltering 6 verbunden und in das Außengehäuse dann das elastische Innengehäuse 2a eingeschoben werden. In jedem Fall schützt das Außengehäuse 11 das flexible Innengehäuse 2a vor unbeabsichtigt aufgebrachten äußeren Druckkräften und hält den Trichterschlitz S geschlossen, der sich nicht öffnen läßt.

Das Außengehäuse 11 ist so ausgebildet, dass der trichterförmigen Kanüleneintrittsöffnung 8 des Innengehäuses 2a fluchtend eine Zugangsöffnung 9 vorgelagert ist. Diese wird von Außengehäuse-Kopfstegen 10a, 10b gebildet, die die Lamellen 8a, 8b des Innengehäuses 2a bzw. dessen Seitenwände 7a, 7b nach oben überkragen und mit ihren Stirnkanten einander zugewandt ausgeformt sind. Am Fuß dieses Überkragungsbereichs sind bei der dargestellten Ausführungsform an den Außengehäuse-Kopfstegen 10a, 10b Innenkonturen 12a, 12b mit einer daran ausgebildeten Schrägfläche 13a bzw. 13b, alternativ ein Radius, vorgesehen, die an komplementären Schrägflächen 14a, 14b an den freien Enden der Seitenwände 7a, 7b des Innengehäuses 2a anliegen. Eine auf das Außengehäuse 11 wirkende Druckkraft wird über diese Schrägflächen 13a, 13b und 14a, 14b und/oder die Seitenwände 7a und 7b definiert in die flexiblen Lamellen 8a, 8b eingeleitet. Ein Öffnen des Trichterschlitzes S wird somit verhindert. Außerdem bieten die Flächen 13a, 13b und 14a, 14b eine Führung beim Ineinanderschieben zum konzentrischen Zusammenbau von Außen- und Innengehäuse 11 bzw. 2a. Das trifft so gleichermaßen bei der in Fig. 5 dargestellten Ausführung des Schutzgehäuses 102 zu, die sich von der vorbeschriebenen Bauweise lediglich dadurch unterscheidet, daß nur die eine Lamelle 8a flexibel ist, während die andere Lamelle 8b des gleichwohl unverändert insgesamt flexiblen Lamellenpaares starr ausgelegt ist.

## Patentansprüche

1. Kanüle, vorzugsweise mit einem Kanülenhalter (1) für insbesondere eine Blutentnahmevorrichtung, die ein schwenkbares, bis auf eine zwischen einander gegenüberliegenden Seitenwänden (7a, 7b) vorgesehene, von mindestens einem an den Seitenwänden (7a, 7b) ausgeformten, nach innen trichterförmig aufeinander zu laufenden, dort einen aufweitbaren Trichterschlitz (S) bildenden Lamellenpaar (8a, 8b) bereitgestellte Kanüleneintrittsöffnung (8) umfangsgeschlossenes Schutzgehäuse (102) für ein freies Kanülenende (4 a) aufweist.
**dadurch gekennzeichnet,**
**dass** das Schutzgehäuse (102) aus zwei konzentrisch ineinander gesteckten Gehäuseteilen besteht wobei ein Innengehäuse (2a) das Lamellenpaar (8a, 8b) aufweist und ein das Innengehäuse (2a) umschließendes Außengehäuse (11) als eine in Bezug auf das Innengehäuse (2a) weniger elastische Hülse ausgebildet ist.

2. Kanüle nach Anspruch 1,
**dadurch gekennzeichnet,**
**dass** das Außengehäuse (11) mit Kontaktflächen (13, 13b) versehen ist, die bei äußerem Druck auf gegenüberliegende, komplementär vorgesehene Kontaktflächen (14a, 14b) des Innengehäuses (2a) eine auf den Trichterschlitz (S) gerichtete Schließkraft einleiten.

## Claims

1. A cannula with a cannula holder (1), more particularly for a blood sampling device, comprising a swivellable protective housing (102), which is circumferentially closed, except for a cannula inlet opening (8) provided between two opposite side walls (7a, 7b), formed by a lamella pair (8a, 8b) converging inwards in funnel-like manner and forming a funnel slit (S), formed on at least one of the side walls (7a, 7b), for a free cannula end (4a)
**characterised in**
**that** the protective housing (102) consists of housing sections inserted into each other, wherein an inner housing (2a) has the lamella pair (8a, 8b) and an outer housing (11) surrounding the inner housing (2a) is designed as a less elastic sleeve in relation to the inner housing (2a).

2. The cannula according to claim 1,
**characterised in**
**that** the outer housing (11) is provided with contact surfaces (13, 13b), which, in the event of pressure on opposite, complementarily provided contact surfaces (14a, 14b) of the inner housing (2a), exert a closing force directed at the funnel slit (S).

## Revendications

1. Canule, de préférence équipée d'un porte-canule (1), en particulier pour dispositif de prise de sang, qui présente un boîtier protecteur pivotable à circonférence fermée (102) prévu sauf à un orifice d'entrée de canule (8) prévu entre des parois latérales opposées (7a, 7b) et constitué par au moins une paire de lamelles (8a, 8b) saillant de parois latérales opposées (7a, 7b), convergeant les unes vers les autres en forme d'entonnoir et y formant une fente d'entonnoir dilatable (S) pour une extrémité de canule libre (4a),
**caractérisée en ce**
**que** le boîtier protecteur (102) est constitué de deux parties de boîtier fichées concentriquement l'une dans l'autre, un boîtier intérieur (2a) comportant la paire de lamelles (8a, 8b) et un boîtier extérieur (11) circonscrivant le boîtier intérieur (2a) sous forme d' un manchon moins élastique que le boîtier intérieur (2a).

2. Canule selon la revendication 1,
**caractérisée en ce**
**que** le boîtier extérieur (11) est pourvu de surfaces de contact (13, 13b) qui, si on exerce une pression extérieure sur des surfaces de contact opposées de type complémentaire (14a, 14b) du boîtier intérieur (2a), induisent une force de fermeture dirigée vers la fente d'entonnoir (S).
